# EUROPÄISCHE PATENTSCHRIFT

(11) **EP 1 034 740 B1**
(45) Veröffentlichungstag und Bekanntmachung des Hinweises auf die Patenterteilung: **16.06.2004**
(21) Anmeldenummer: 00102503.0
(22) Anmeldetag: 05.02.2000
(51) Int. Cl.: A61B 5/15

(54) **Gerät zur Entnahme von Blut für Diagnosezwecke**
Apparatus for taking blood for diagnostic purposes
Appareil de prélèvement de sang à but diagnostique

(30) Priorität: 05.03.1999 DE 19909602
(43) Veröffentlichungstag der Anmeldung: 13.09.2000
(73) Patentinhaber: Roche Diagnostics GmbH, 68298 Mannheim (DE)
(72) Erfinder: Kuhr, Hans J-rgen, 68219 Mannheim (DE); Forster, Richard, 92536 Pfreimd (DE)
(74) Vertreter: Pfeifer, Hans-Peter, Dr.

(56) Entgegenhaltungen:
- EP-A- 0 565 970

## Beschreibung

Die Erfindung betrifft ein Blutlanzettengerät zur Entnahme von Blut für Diagnosezwecke.

Um für Diagnosezwecke eine geringe Menge Blut aus einem Körperteil (üblicherweise dem Finger oder dem Ohrläppchen) zu entnehmen, werden Lanzetten verwendet, die in das entsprechende Körperteil gestochen werden. Das Einstechen erfolgte in der Vergangenheit durch trainiertes, speziell ausgebildetes Personal manuell oder mit einer einfachen Apparatur. Soweit die Blutuntersuchungen nur in relativ großen zeitlichen Abständen durchgeführt werden müssen, ist diese Form der Blutgewinnung akzeptabel, weil der Schmerz, der mit dem Einstechen verbunden ist, dabei keine entscheidende Polle spielt.

Wesentlich höhere Anforderungen sind an die Blutentnahme zu stellen, wenn eine regelmäßige Überwachung bestimmter analytischer Werte des Blutes von Patienten erforderlich ist. Dies gilt insbesondere für Diabetiker, die ihren Blutzuckerspiegel häufig und regelmäßig kontrollieren sollten, um durch Anpassung von Insulininjektionen an den Bedarf (der von der Nahrungsaufnahme, der körperlichen Aktivität und anderen Faktoren abhängt) ihren Blutzuckerspiegel möglichst ständig innerhalb bestimmter Sollgrenzen zu halten. Diese Blutzucker-Intensivtherapie ist für die Gesundheit der Patienten von größter Bedeutung und erfordert mindestens vier Blutentnahmen pro Tag. Beispielsweise wird in der Publikation
"The effect of intensive treatment of diabetes on the development and progression of long-term complications in insulin-dependent diabetes mellitus" der Diabetes Control and Complications Trial Research Group, publiziert in The New England Journal of Medicine, 1993, 977 bis 986
berichtet, daß durch eine Intensivtherapie mit mindestens vier Blutentnahmen pro Tag ein Rückgang der letztlich zur Erblindung des Patienten führenden Retinopathie um 76 % erreicht werden kann. Ähnliches gilt für andere schwere Spätschäden des Diabetes mellitus.

Die Blutzucker-Intensivtherapie ist in der Praxis längerfristig nur im Rahmen des sogenannten "home-monitoring", also durch den Patienten oder dessen Angehörige ohne trainiertes medizinisches Personal, möglich. Dabei ist die Bereitschaft und Fähigkeit des Patienten, mindestens viermal täglich eine Blutentnahme mittels einer Lanzette durchzuführen, ganz entscheidend von den Eigenschaften des Blutentnahmegerätes abhängig. Es muß so konstruiert sein, daß der Schmerz bei der Erzeugung der für die Blutentnahme erforderlichen Wunde möglichst gering ist. Die Handhabung des Gerätes muß möglichst einfach sein, insbesondere weil viele der betroffenen Patienten wegen ihrer Erkrankung oder wegen fortgeschrittenen Alters nicht in der Lage sind, diffizile Handhabungsvorgänge präzise auszuführen. Weiterhin ist ein geringes Gewicht und eine praktische Form wichtig, um das Gerät ohne wesentliche Belastung ständig mitführen zu können. Schließlich soll die Konstruktion möglichst einfach, langlebig und kostengünstig sein.

Um diesen Anforderungen gerecht zu werden, wurden bereits eine Vielzahl von Gestaltungen und Konstruktionen von Blutentnahmegeräten und zugehörigen Lanzetten vorgeschlagen, die beispielsweise in den US-Patenten
4,442,836
4,535,769
4,469,110
4,653,513
4,895,147
4,924,879
5,318,584
5,554,166
beschrieben sind.

Obwohl durch diese Konstruktionen wesentliche Verbesserungen bei der Blutentnahme für Diagnosezwecke erreicht werden konnten, können sie hinsichtlich der Gesamtheit der vorgenannten Anforderungen nicht in vollem Umfang befriedigen. Insbesondere sind bei denjenigen Geräten, bei denen die Erzeugung der Wunde mit sehr geringen Schmerzen verbunden ist, Nachteile hinsichtlich der Handhabung und/ oder Geräteform- und größe festzustellen. Umgekehrt wird mit besonders kleinen und einfach bedienbaren Geräten keine befriedigende Schmerzarmut erreicht.

Der Erfindung liegt auf dieser Basis die Aufgabe zugrunde, ein verbessertes Blutentnahmegerät zu schaffen, das die Gesamtheit der genannten Anforderungen besser als bisher erfüllt.

Die Aufgabe wird gelöst durch ein Gerät zur Entnahme von Blut für Diagnosezwecke, mit einem langgestreckten Gehäuse, an dessen Vorderende eine Austrittsöffnung für die Spitze einer Lanzette vorgesehen ist, einem in dem Gehäuse in Richtung von dessen Hauptachse entlang einem vorbestimmten Einstichweg beweglichen Lanzettenhalter zur Halterung der Lanzette, einer Lanzettenführung zur Führung des Lanzettenhalters auf dem vorbestimmten Einstichweg und einem Lanzettenantrieb mit einer elastischen Antriebsfeder, der im gespannten Zustand der Antriebsfeder mittels einer Arretiervorrichtung arretierbar ist und durch den nach dem Lösen der Arretiervorrichtung die Entspannungsbewegung der Antriebsfeder in eine Einstichbewegung umgesetzt wird, bei der die von dem Lanzettenhalter gehaltene Lanzette mit hoher Geschwindigkeit entlang dem vorbestimmten Einstichweg in Einstichrichtung bewegt wird, bis ihre Spitze aus der Austrittsöffnung austritt, um in einem an der Austrittsöffnung anliegenden Körperteil eine Wunde zu erzeugen, bei welchem in dem Gehäuse ein doppelseitiges Drehschiebegetriebe vorgesehen ist, auf dessen Eingangsseite die Bewegung eines aus dem Gehäuse an dessen Hinterende herausragenden Spannknopfes längs eines linearen Spannweges in eine Drehbewegung eines Lanzettenantriebsrotors umgesetzt wird, der um eine zu der Geräteachse parallel verlaufende Rotationsachse gedreht wird, um den Lanzettenantriebsrotor gegen die Kraft der Antriebsfeder zu spannen und auf dessen Ausgangsseite nach dem Auslösen des Lanzettenantriebs eine durch die Antriebsfeder angetriebene Rotationsbewegung des Lanzettenantriebsrotors in die Einstichbewegung in Richtung der Hauptachse umgesetzt wird.

Die Erfindung richtet sich auch auf einen Blutentnahme-Kit, der als aufeinander abgestimmte Systembestandteile ein erfindungsgemäßes Blutentnahmegerät und Lanzetten umfaßt, die zur Halterung in dem Lanzettenhalter des Gerätes ausgebildet sind. Solche Blutentnahme-Kits (die man auch als Blutentnahme-Ausrüstung bezeichnen kann) werden (als Erstausrüstung) in Form von Verpackungseinheiten verkauft, die beide Systembestandteile enthalten. Da die Lanzetten üblicherweise nur einmal verwendbar sind, werden sie für den weiteren Gebrauch auch in gesonderter Verpackung zur Verwendung mit einem bei dem Benutzer bereits vorhandenen Blutentnahmegerät angeboten.

Der Begriff "Getriebe" wird hier im allgemeinen Sinn verstanden, d.h. als eine kinematische Vorrichtung, die zur Kopplung und Umwandlung von Bewegungen dient. Im vorliegenden Fall wird durch das doppelseitige Drehschiebegetriebe einerseits eine translatorische Bewegung des Spannknopfes in eine Rotationsbewegung des Lanzettenantriebsrotors und andererseits eine Rotationsbewegung des Lanzettenantriebsrotors in eine Translationsbewegung des Lanzettenhalters mit der Lanzette transformiert. Diese Getriebefunktionen können grundsätzlich mit Hilfe von aus der Maschinenkonstruktionslehre bekannten Konstrukionselementen realisiert werden.

Durch die Erfindung werden unter anderem folgende Vorteile erreicht:
- Die Konstruktion ermöglicht eine sehr schlanke Gehäuseform ähnlich einem Kugelschreiber (sogenannte "pencil-form"). Das Gerät ist dadurch unauffällig und leicht transportabel.
- Das Gerät kann hinsichtlich des Spann- und hinsichtlich des Auslösevorgangs mit nur einer Hand bedient werden.
- Die Handhabung ist leicht und intuitiv.
- Der Schmerz bei der Erzeugung der Wunde ist sehr gering. Dies ist teilweise darauf zurückzuführen, daß eine sehr gute Vibrationsarmut erreicht wird.
- Trotz der wesentlichen Verbesserung der Funktion ist die Konstruktion einfach und kostengünstig.

Bei der Realisierung der Erfindung sind besondere Probleme zu berücksichtigen, die vor allem daraus resultieren, daß ein schmerzarmer Einstich eine sehr schnelle und präzise geführte Einstichbewegung voraussetzt. Um diese zu ermöglichen, muß die Antriebsfeder eine hohe Federkraft ausüben. Andererseits soll die Betätigung des Spannknopfes so leicht sein, daß auch ältere und körperlich eingeschränkte Personen das Blutentnahmegerät problemlos spannen können.

Diese Probleme werden besonders gut gelöst und die oben genannten Vorteile werden in besonderem Maße erreicht, wenn die in der nachfolgenden Beschreibung und in den Unteransprüchen genannten Merkmale bevorzugter Ausgestaltungen realisiert werden. Diese Merkmale können einzeln oder in Kombination bei dem erfindungsgemäßen Blutentnahmegerät vorhanden sein.

Gemäß einer bevorzugten Ausführungsform weist der Lanzettenantriebsrotor eine schraubenförmig als Wendel verlaufende Gleitfläche und der Spannknopf einen Spannocken auf, der mittels einer Kontaktfläche auf der Oberfläche der Wendel gleitet, um eine Linearbewegung des Spannknopfes in eine Rotationsbewegung des Lanzettenantriebsrotors umzusetzen. Dabei ist vorzugsweise die Wendel an einer Spannhülse ausgebildet, die einen Teil des Antriebsrotors bildet und deren der Austrittsöffnung zugewandtes vorderes Ende den Lanzettenhalter umgibt.

Eine besonders einfache Konstruktion und vibrationsarme Funktion wird erreicht, wenn gemäß einer weiteren bevorzugten Ausführungsform an dem vorderen Ende der Spannhülse eine Steuerkurve vorgesehen ist, die im Zusammenwirken mit einem an dem Lanzettenhalter vorgesehenen Steuerzapfen die Einstichbewegung und bevorzugt auch die Rückführbewegung des Lanzettenhalters und der darin gehaltenen Lanzette steuert. Die Steuerung der Einstichund Rückführbewegung durch das Zusammenwirken eines Steuerzapfens und einer Steuerkurve ist aus den oben erwähnten US-Patentschriften 5,318,584 und 5,554,106 bekannt.

Die Erfindung wird nachfolgend anhand eines in den Figuren dargestellten Ausführungsbeispieles näher erläutert; es zeigen:
- Fig. 1: eine Prinzipdarstellung eines Blutentnahmegerätes im Querschnitt,
- Fig. 2: eine Seitenansicht eines Blutentnahmegerätes,
- Fig. 3: einen Querschnitt des Blutentnahmegerätes von Figur 2 mit in den Lanzettenhalter eingesetzter Lanzette,
- Fig. 4: eine Explosionsdarstellung der für die Funktion gemäß der vorliegenden Erfindung wesentlichen Bauteile eines Blutentnahmegerätes,
- Fig. 5: eine Detaildarstellung des hinteren Endes des in Figur 4 dargestellten Lanzettenantriebsrotors und des in Figur 4 dargestellten Spannknopfes, wobei letzterer aufgeschnitten ist,
- Fig. 6: eine Abwicklung der Wendel der in den Figuren 2 und 3 dargestellten Spannhülse in eine Ebene.
- Fig. 7: eine erste aufgeschnittene Ansicht einer in Figur 4 dargestellten Spannhülse mit darin eingesetzter Kurvenhülse, und
- Fig. 8: eine zweite aufgeschnittene Ansicht der Spannund Kurvenhülse von Figur 7.

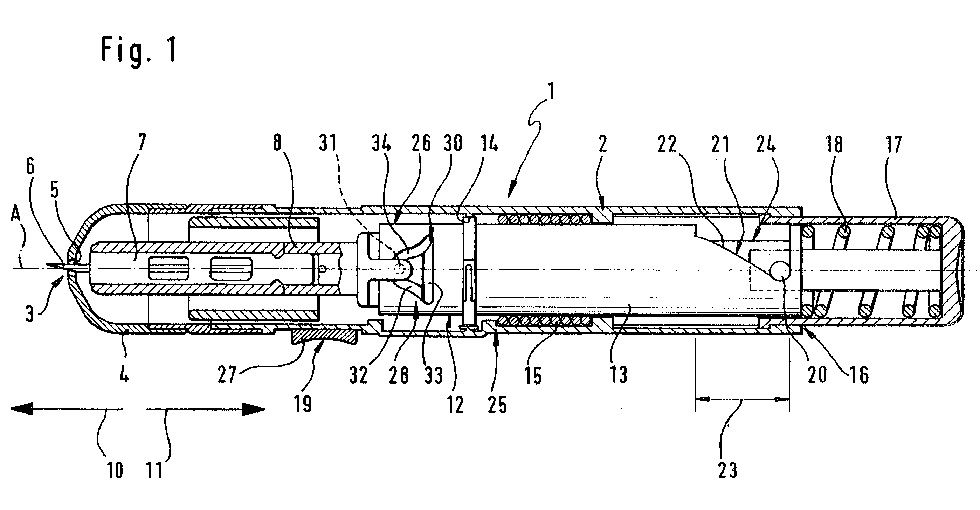

Das in Figur 1 in Form einer schematischen Prinzipdarstellung gezeigte Blutentnahmegerät 1 hat ein langgestrecktes Gehäuse 2, das sich längs der Geräte-Hauptachse A erstreckt. Das Vorderende 3 des Gehäuses 2 wird von einer abnehmbaren Kappe 4 gebildet, in der eine Austrittsöffnung 5 für die Spitze 6 einer Lanzette 7 vorgesehen ist.

Die Lanzette 7 wird von einem Lanzettenhalter 8 so gehalten, daß sich die Spitze 6 bezogen auf den Halter 8 auch dann reproduzierbar in der gleichen Position befindet, wenn für aufeinanderfolgende Blutentnahmen jeweils eine neue Lanzette 7 in den Halter 8 eingesetzt wird. Vorteilhafte Maßnahmen, um dies zu erreichen, die auch für die vorliegende Erfindung verwendet werden können, sind in dem US-Patent 5,318,584 beschrieben.

Zum Erzeugen einer Wunde wird das Vorderende 3 des Gehäuses 2 gegen ein Körperteil gedrückt, in dem eine Wunde zur Gewinnung eines Blutstropfens erzeugt werden soll. Danach führt der Lanzettenhalter 8 eine Einstichbewegung aus, bei der die von dem Lanzettenhalter 8 gehaltene Lanzette 7 mit hoher Geschwindigkeit entlang einem vorzugsweise längs der Hauptachse A verlaufenden vorbestimmten Einstichweg in die durch den Pfeil 10 symbolisierte Einstichrichtung bewegt wird, bis ihre Spitze 6 aus der Austrittsöffnung 5 austritt und in das Körperteil eindringt.

Danach wird die Lanzette 7 in der durch den Pfeil 11 symbolisierten Rückführungsrichtung in die Ausgangslage zurückbewegt.

Die Einstich- und Rückführungsbewegung des Lanzettenhalters 8 (und somit der Lanzette 7) wird durch einen insgesamt mit 12 bezeichneten Lanzettenantrieb angetrieben, dessen zentrales Element ein Lanzettenantriebsrotor 13 ist, der in dem Gehäuse 2 mittels eines Rotationslagers 14 in einer festen Axialposition, jedoch um die Hauptachse A rotierbar, gelagert ist. Der Lanzettenantriebsrotor 13 steht unter der Wirkung einer Antriebsfeder 15, die als den Antriebsrotor 14 umgebende Torsionsfeder ausgebildet ist. Ein Ende der Antriebsfeder 15 ist an dem Lanzettenantriebsrotor 13, das andere Ende an dem Gehäuse 2 befestigt.

Die Antriebsfeder 15 wird durch eine ihrer Federkraft entgegenwirkende Drehung des Lanzettenantriebsrotors 13 gespannt. Im dargestellten Fall ist hierzu - betrachtet vom Hinterende 16 des Gehäuses - eine Linksdrehung des Rotors 13 erforderlich. Diese Spannbewegung wird dadurch bewirkt, daß ein aus dem Gehäuse 2 an dessen Hinterende 16 herausragender, in der Gegenrichtung von einer Rückstellfeder 18 belasteter Spannknopf 17 längs der Hauptachse A in Richtung auf das Vorderende 3 bewegt wird. Dabei gleitet ein mit dem Spannknopf 17 fest verbundener Spannocken 20 über eine an dem Antriebsrotor 13 ausgebildete in Form einer schraubenförmigen Wendel 21 verlaufende Gleitfläche 22. Der Spannknopf 17 und mit ihm der Spannocken 20 sind axial verschiebbar, jedoch rotationsfest gelagert. Dies führt in Verbindung mit der Form der Wendel 21 dazu, daß die Bewegung des Spannknopfes 17 längs eines linearen durch den Pfeil 23 symbolisierten Spannweges in eine Rotationsbewegung des Lanzettenantriebsrotors um eine zu der Hauptachse parallel verlaufende Rotationsachse umgesetzt wird, um den Lanzettenantriebsrotor 13 gegen die Kraft der Antriebsfeder 15 zu spannen. Nach dem Spannvorgang wird der Lanzettenantriebsrotor mittels einer in Figur 1 nicht detailliert dargestellten Arretiervorrichtung 19 im gespannten Zustand arretiert. Der Spannknopf 17 mit dem Nocken 20 und der Antriebsrotor 13 mit der als Wendel 21 ausgebildeten Gleitfläche 22 bilden die insgesamt mit 24 bezeichnete Eingangsseite eines doppelseitigen Drehschiebegetriebes 25.

Die Ausgangsseite 26 des doppelseitigen Drehschiebegetriebes 25 ist so ausgebildet, daß nach dem Auslösen des Lanzettenantriebs mittels eines Auslöseknopfes 27 (dessen Funktion in Figur 1 nicht dargestellt ist) eine durch die Antriebsfeder 15 angetriebene Rotationsbewegung des Lanzettenantriebsrotors 13 in die Einstichbewegung 10 in Richtung der Hauptachse A umgesetzt wird. Zu diesem Zweck ist eine insgesamt mit 28 bezeichnete Kurvensteuerung vorgesehen, die durch eine Steuerkurve 30 und einen in die Steuerkurve 30 passenden Steuerzapfen 31 gebildet ist. Der Steuerzapfen 31 ist mit dem Lanzettenhalter 8 fest verbunden, der seinerseits axial verschiebbar aber rotationsfest gelagert ist. Die Steuerkurve 30 wird von einer Ausnehmung 32 des Lanzettenantriebsrotors 13 gebildet. Sie ist so gestaltet, daß der Steuerzapfen 31 während der Spannbewegung einen ersten Abschnitt 33 der Steuerkurve 30 abfährt, der bei der dargestellten Ausführungsform im wesentlichen gerade und quer zu der Hauptachse A verläuft. Während der Einstich- und Rückführbewegung fährt der Steuerzapfen 31 einen zweiten Abschnitt 34 der Steuerkurve 30 ab, der zunächst in Richtung des Vorderendes 3 und dann in Richtung des Hinterendes 16 verläuft und dadurch eine definierte Einstich- und Rückführbewegung des Halters 8 bewirkt. Nähere Einzelheiten einer derartigen Konstruktion sind der erwähnten US-Patentschrift 5,318,584 zu entnehmen. Während bei der dort dargestellten Ausführungsform die Steuerkurve ein Bestandteil des Lanzettenhalters ist und der Steuerzapfen rotiert, ist es bei der vorliegenden Erfindung - wie dargestellt - vorzugsweise umgekehrt: Der Steuerzapfen ist mit dem Lanzettenhalter 8 fest verbunden und axial beweglich, während die Steuerkurve 30 mit dem Lanzettenantriebsrotor 13 rotiert.

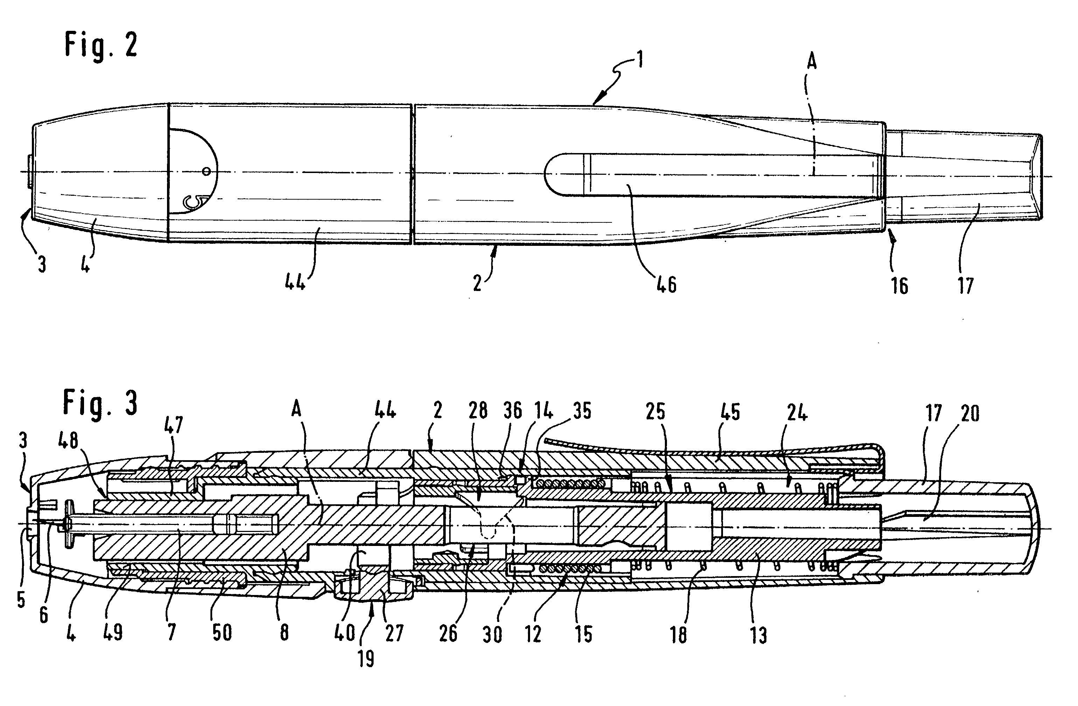

Die Figuren 2 bis 8 zeigen unterschiedliche Darstellungen einer besonders vorteilhaften praktischen Ausführungsform eines erfindungsgemäßen Blutentnahmegerätes. Es handelt sich dabei jeweils um in sich maßstäbliche Darstellungen, d.h. die Proportionen der Bauteile zueinander entsprechen in jeder der Figuren den tatsächlichen Verhältnissen. Die in Zusammenhang mit Figur 1 beschriebenen Bauteile sind in den Figuren 2 bis 8 mit den gleichen Bezugszeichen gekennzeichnet und werden nicht nochmals beschrieben.

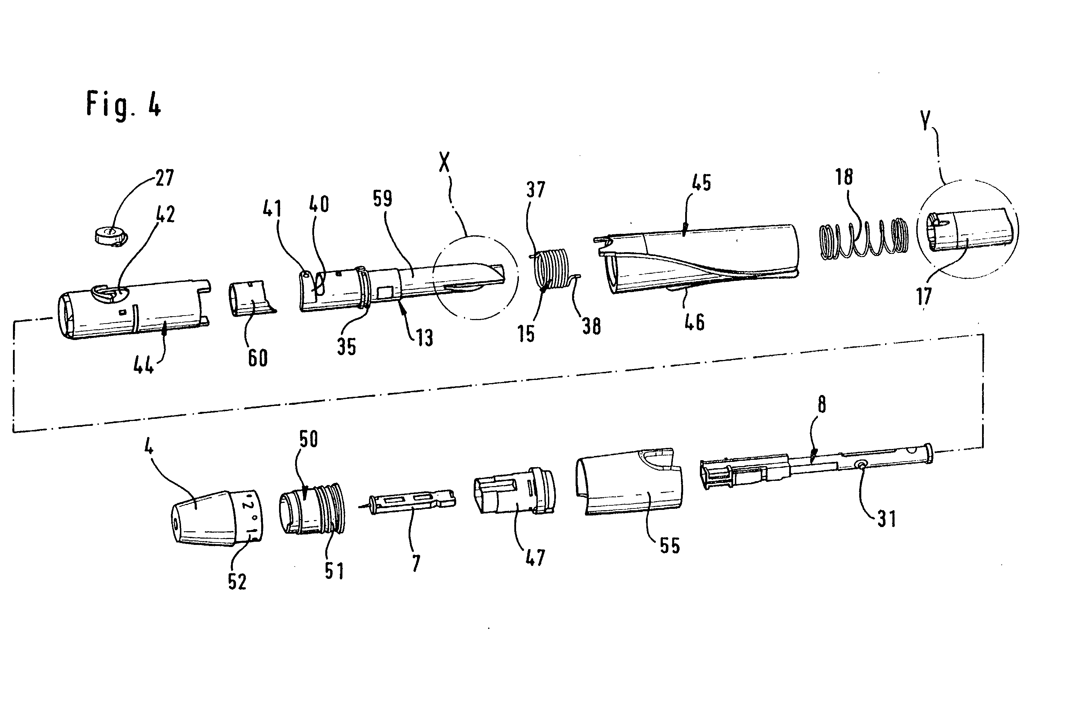

In den Figuren 3 und 4 ist zu erkennen, daß das Rotationslager 14 zweckmäßigerweise durch einen an dem Lanzettenantriebsrotor 13 ausgebildeten von dessen Umfangsfläche vorspringenden Lagerring 35 und einen korrespondierenden Absatz 36 des Gehäuses 2 gebildet wird. Der Lagerring 35 weist eine Unterbrechung auf, in die ein erster Schenkel 37 der Antriebsfeder 15 eingreift. Der zweite Schenkel 38 ist an dem Gehäuse 2 fixiert.

In den Figuren 2 bis 4 ist der bevorzugte Aufbau des Gehäuses 2 zu erkennen. Dabei ist der Lanzettenantrieb 12 von zwei axial zusammensteckbaren Gehäuseteilen, nämlich einem vorderen Gehäuseteil 44 und einem hinteren Gehäuseteil 45 umgeben. An dem hinteren Gehäuseteil 45 ist ein Clip 46 angebracht, mit dem das Blutentnahmegerät 1 beispielsweise in einer Sakkotasche befestigt werden kann. In das vordere Gehäuseteil 44 ist ein unteres Einsatzteil 47 eingesetzt, das eine zentrale Axialöffnung 48 aufweist, deren Innenkontur der Außenkontur des Lanzettenhalters 8 entspricht. Die Wände der Axialöffnung 48 bilden eine präzise Führung 49 für die Einstich- und Rückführbewegung des Lanzettenhalters 8.

Das untere Ende des Einsatzteiles 47 ist von einem Einstellring 50 umgeben, auf dessen Außenseite ein Gewinde 51 vorgesehen ist, auf das die Kappe 4 aufgeschraubt wird. Durch Verdrehen der Kappe 4 relativ zu dem Einstellring 50 kann ihre Längsposition bezüglich des Lanzettenhalters 8 variiert und dadurch die Einstichtiefe eingestellt werden. Die Einstellung kann mittels einer an der Kappe 4 angebrachten Skala 52 kontrolliert werden.

Die Arretiervorrichtung 19 ist bei der dargestellten Ausführungsform in der Weise realisiert, daß der Lanzettenantriebsrotor 13 eine Federlasche 40 aufweist, von der ein kurzer Arretierstift 41 radial nach außen vorsteht, der zur Arretierung des Lanzettenantriebs im gespannten Zustand der Feder 15 in eine entsprechende Ausnehmung 42 des Gehäuseteils 44 eingreift. Zum Auslösen wird der Auslöseknopf 27 mit seinem als Auslöser dienenden unteren Ende so in die Ausnehmung 42 gedrückt, daß der Arretierstift 41 frei wird. Der Auslöseknopf 27 ist vorzugsweise durchsichtig, so daß im gespannten Zustand der Arretierstift 41 und möglicherweise auch Teile der Federlasche 40 durch den Auslöseknopf 27 hindurch erkennbar sind. Diese Bauteile sind vorzugsweise mit einer kontrastierenden Farbe (beispielsweise gelb oder rot) eingefärbt. Dadurch ist auf einfache Weise der gespannte Zustand des Blutentnahmegerätes 1 erkennbar.

Das untere Gehäuseteil 44 und die Kappe 4 sind teilweise von einer Auswerferhülse 55 umgeben, mittels der die Lanzette 7 aus dem Halter 8 ausgeworfen werden kann, nachdem zuvor die Kappe 4 einschließlich des Einstellrings 50 am unteren Ende des Gehäuses 2 abgenommen wurde.

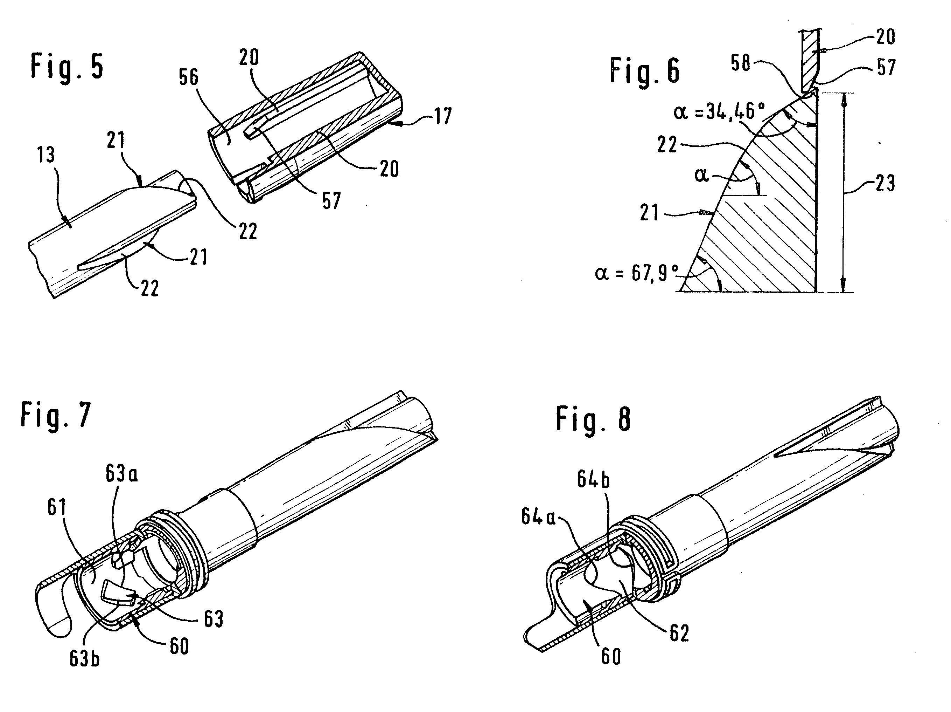

Einzelheiten einer besonders bevorzugten Gestaltung der Eingangsseite 24 des doppelseitigen Drehschiebegetriebes werden durch die Figuren 5 und 6 verdeutlicht.

Wie in Figur 5 zu erkennen ist, sind an dem hinteren Ende des Lanzettenantriebsrotors 13 bevorzugt zwei in Form je einer schraubenförmigen Wendel 21 gestaltete parallel verlaufende Gleitflächen 22 vorgesehen, die mit entsprechenden an der Innenwandung 56 des Spannknopfes 17 angeordneten Spannocken 20 zusammenwirken. Bei dem Spannvorgang gleiten die Spannocken 20 jeweils mit schräg geneigten Kontaktflächen 57 über die Gleitflächen 22 der Wendeln 21.

Dabei ist es besonders vorteilhaft, wenn, wie in Figur 6 am besten zu erkennen ist, die Steigung der Wendel 21 (d.h. der Winkel α zwischen der Gleitfläche und einer zu der Hauptachse senkrechten die Gleitfläche in dem jeweiligen Punkt schneidenden Geraden) über die Länge des Spannweges 23 nicht konstant, sondern variabel ist. Bevorzugt nimmt der Winkel α über die Länge der Wendel (entsprechend der Länge des Spannweges 23) in Richtung auf das Vorderende 3 des Gehäuses 2 mindestens abschnittsweise, besonders bevorzugt kontinuierlich, zu. Beispielsweise beträgt bei dem in Figur 6 dargestellten Ausführungsbeispiel der Winkel α an dem vorderen Ende der Wendel etwa 68° und an dem hinteren Ende etwa 34,5°. Dabei sollte die Steigung etwa derartig variabel sein, daß die zum Spannen erforderliche auf den Spannknopf 17 anzuwendende Kraft über den Spannweg 23 mindestens abschnittsweise im wesentlichen konstant ist. Da die Rückstellkraft der Antriebsfeder 15 während des Spannvorganges zunimmt, kann die Übersetzung der Eingangsseite 24 des Drehschiebegetriebes am Anfang des Spannvorganges relativ groß sein und sollte entlang dem Spannweg 23 kontinuierlich abnehmen, wobei dies durch den zunehmenden Steigungswinkel α erreicht wird.

Die Kontaktfläche 57 des Spannockens 20 ist bevorzugt derartig geneigt, daß mindestens auf einem Teil der dem Vorderende des Gehäuse zugewandten Hälfte der Wendellänge Flächenkontakt zu der Wendel besteht. Bei der in den Figuren dargestellten Ausführungsform stimmt die Neigung der Kontaktfläche 57 mit der Steigung der Gleitfläche 22 in deren vorderem (in Figur 6 unteren) Abschnitt überein. Dadurch wird ein besonders leichtes Gleiten und ein geringer Verschleiß in dem Bereich des Spannvorganges erreicht, in dem die höchste Gegenkraft der Feder 15 wirkt.

Um die Belastungen beim Auftreffen eines derartig ausgebildeten Spannockens 20 auf die Gleitfläche 22 der Wendel 21 zu vermindern, ist an dem dem hinteren Ende 16 des Gehäuses 2 zugewandten Ende der Wendel 21 ein rampenförmiger Startabschnitt 58 vorgesehen, dessen Steigung der Neigung der Gleitfläche entspricht. Obwohl am unteren Ende des rampenförmigen Startabschnittes 58 kurzzeitig die Kontaktfläche des Spannockens 20 nur auf einer schmalen Linie berührt, ergibt sich in diesem Bereich wegen der geringen Federkraft kein überhöhter Verschleiß. Vielmehr ist die Bedienung bei Realisierung der bevorzugten Ausführungsformen außerordentlich leicht.

Im Hinblick auf eine leichte Bedienbarkeit ist auch die Auswahl des für den Lanzettenantriebsrotor 13 (zumindest dessen Gleitfläche 22) und den Spannocken 20 (zumindest dessen Kontaktfläche 57) verwendeten Materials wesentlich. Bevorzugt wird für erstere ein Kunststoff auf Basis eines Polyacetals, insbesondere auf Basis eines Polyoxymethylens (POM) verwendet, während für letztere ein Kunststoff auf Basis eines Styrols-Acrylnitril-Copolymers (SAN) besonders geeignet ist.

In den Figuren 3, 4, 7 und 8 ist zu erkennen, daß der Lanzettenantriebsrotor 13 aus zwei Teilen, nämlich einer Spannhülse 59 und einem in diese von vorne her einsetzbare Kurvenhülse 60 besteht. Das vordere Ende der Spannhülse 59 mit der darin eingesetzten Kurvenhülse 60 umschließt das hintere Ende des Lanzettenhalters 8 in dem Bereich, in dem dieser zwei diametral gegenüberliegende Steuerzapfen 31 aufweist. Durch die innenseitige Profilierung der Spannhülse 59 und der darin eingesetzten Kurvenhülse 60 werden zwei Ausnehmungen des Lanzettenantriebsrotors 13 gebildet, deren Begrenzungskanten gemeinsam eine Steuerkurve definieren. Die Ausnehmungen 61 und 62 sind so ausgebildet, daß in jeder Phase der Rotationsbewegung eine der Ausnehmungen 61 die Führung des einen Steuerzapfens nach hinten und die andere Ausnehmung 62 die Führung des Steuerzapfens nach vorne bildet.

Im dargestellten Fall bildet das in Figur 7 dargestellte erhabene Flächenstück 63 mit seiner hinteren Kante 63a eine Begrenzung für die Bewegung eines Steuerzapfens 31 während der Rückführungsphase nach vorne hin, d.h. die Bewegungsmöglichkeit des Lanzettenhalters 8 nach vorne wird durch die hinteren Kanten 63a des erhabenen Flächenstücks 63 begrenzt. Entsprechend begrenzen die nach vorne gewandten Kanten 63b des erhabenen Flächenstücks 63 die Bewegungsmöglichkeit des Lanzettenhalters 8 während der Einstich- und Rückführungsphase nach hinten.

Die Bewegungsmöglichkeit des Lanzettenhalters 8 während der Einstich- und Rückführungsphase nach vorne wird durch die in Figur 8 erkennbare obere Kante 64a der Kurvenhülse 60 begrenzt. Die ebenfalls in Figur 8 erkennbare vordere Kante 64b, die in der Spannhülse 59 ausgebildet ist, begrenzt die Bewegung des Lanzettenhalters 8 während des Spannvorganges nach hinten.

Insgesamt bilden also die Begrenzungen 63a, 63b, 64a, 64b der an der Innenwandung des Lanzettenantriebsrotors 13 gebildeten Ausnehmungen 61 und 62 eine gemeinsame Steuerkurve, die von den beiden Steuerzapfen 31 abgefahren wird. Durch diese bevorzugte Ausführungsform ist eine extrem schlanke Bauweise möglich.

Bei dem dargestellten Blutentnahmegerät ist sowohl die Wendel-Gleitfläche 22 als auch die Steuerkurve 30 an einem einheitlich starren (lediglich aus Herstellungsgründen aus der Spannhülse 59 und der Kurvenhülse 60 bestehenden) Bauteil ausgebildet. Obwohl eine solche starre Konstruktion des Antriebsrotors 13 bevorzugt ist, ist grundsätzlich auch eine Gestaltung möglich, bei der die erforderliche Rotationskopplung zwischen der Steuerkurve 30 und der Wendel-Gleitfläche 22 indirekt, beispielsweise über eine Verbindungsstange bewirkt wird. Als Antriebsrotor im Sinne der Erfindung ist stets die um die Hauptachse rotierende Einheit zu verstehen, unabhängig davon, ob sie aus einem oder mehreren Bauteilen besteht.

Der Spannocken 20 ist bevorzugt, wie dargestellt, an einem einstückigen Bauteil ausgebildet, das den Spannknopf 17 bildet. Auch dies ist nicht zwingend notwendig. Es sind auch Konstruktionen möglich, bei denen der Spannokken 20 und der Auslöseknopf 16 an getrennten Bauteilen vorgesehen sind, die allerdings derartig miteinander verbunden sein müssen, daß der Spannocken 20 bei der Betätigung des Spannknopfes 17 synchron mit diesem entlang des linearen Spannweges 23 bewegt wird.

Das erfindungsgemäße Blutentnahmegerät kann insgesamt sehr schlank gestaltet sein. Der größte Durchmesser des Blutentnahmegerätes sollte jedenfalls unter 20 mm liegen. Auch ein Wert von weniger als 15 mm ist bei Verwendung der erfindungsgemäßen Konstruktion möglich.

Diese schlanke Bauform ist nicht nur im Hinblick auf die leichte Transportierbarkeit des Blutentnahmegerätes wichtig. Für die mit der Erfindung erreichten Vorteile ist vielmehr auch wichtig, daß sie zu einem sehr kleinen Rotations-Trägheitsmoment des Lanzettenantriebsrotors führt. Infolge dieses geringen Rotations-Trägheitsmomentes kann mit einer verhältnismäßig schwachen Antriebsfeder, deren Federkonstante etwa zwischen 25 und 35 Nmm liegt, bei einem Spannweg von 15 mm der Lanzettenantrieb mit einer Spannkraft von nur etwa 11 Newton gespannt werden. Trotz dieser leichten Bedienbarkeit wird eine außerordentlich schnelle und präzise Lanzettenbewegung bei geringer Vibration des Gerätes erreicht. Hieraus resultiert ein minimales Schmerzempfinden.

## Patentansprüche

1. Blutentnahmegerät zur Entnahme von Blut für Diagnosezwecke, mit
einem langgestreckten Gehäuse (2), an dessen Vorderende (3) eine Austrittsöffnung (5) für die Spitze (6) einer Lanzette (7) vorgesehen ist,
einem in dem Gehäuse (2) in Richtung von dessen Hauptachse (A) entlang einem vorbestimmten Einstichweg beweglichen Lanzettenhalter (8) zur Halterung der Lanzette (7),
einer Lanzettenführung (49) zur Führung des Lanzettenhalters (8) auf dem vorbestimmten Einstichweg und
einem Lanzettenantrieb (12) mit einer elastischen Antriebsfeder (15), der im gespannten Zustand der Antriebsfeder (15) mittels einer Arretiervorrichtung (19) arretierbar ist und durch den nach dem Lösen der Arretiervorrichtung die Entspannungsbewegung der Antriebsfeder (15) in eine Einstichbewegung umgesetzt wird, bei der die von dem Lanzettenhalter (8) gehaltene Lanzette (7) mit hoher Geschwindigkeit entlang dem vorbestimmten Einstichweg in Einstichrichtung (10) bewegt wird, bis ihre Spitze (6) aus der Austrittsöffnung (5) austritt, um in einem an der Austrittsöffnung (5) anliegenden Körperteil eine Wunde zu erzeugen,
**dadurch gekennzeichnet, daß**
in dem Gehäuse (2) ein doppelseitiges Drehschiebegetriebe (25) vorgesehen ist,
a) auf dessen Eingangsseite (24) die Bewegung eines aus dem Gehäuse (2) an dessen Hinterende (16) herausragenden Spannknopfes (17) längs eines linearen Spannweges (23) in eine Drehbewegung eines Lanzettenantriebsrotors (13) umgesetzt wird, der um eine zu der Geräteachse (A) parallel verlaufende Rotationsachse gedreht wird, um den Lanzettenantriebsrotor (13) gegen die Kraft der Antriebsfeder (15) zu spannen und
b) auf dessen Ausgangsseite (26) nach dem Auslösen des Lanzettenantriebs eine durch die Antriebsfeder angetriebene Rotationsbewegung des Lanzettenantriebsrotors in die Einstichbewegung in Richtung der Hauptachse umgesetzt wird.

2. Blutentnahmegerät nach Anspruch 1, bei welchem die Eingangsseite (24) des doppelseitigen Drehschiebegetriebes (25) mittels einer an dem Lanzettenantriebsrotor (13) vorgesehenen Wendel (21) und eines mit dem Spannknopf (17) verbundenen mit diesem längs des Spannweges (23) beweglichen und auf einer Gleitfläche (22) der Wendel (21) mittels einer Kontaktfläche (57) gleitbaren Spannockens (20) ausgebildet ist.

3. Blutentnahmegerät nach Anspruch 2, bei welchem die Steigung (α) der Wendel (21) über deren Länge in Richtung auf das Vorderende (3) des Gehäuses (2) mindestens abschnittsweise zunimmt.

4. Blutentnahmegerät nach Anspruch 3, bei welchem die Steigung (α) derartig variabel ist, daß die zum Spannen des Lanzettenantriebs (12) erforderliche auf den Spannknopf (17) anzuwendende Spannkraft über den Spannweg (23) wenigstens abschnittsweise im wesentlichen konstant ist.

5. Blutentnahmegerät nach einem der Ansprüche 2 bis 4, bei welchem die Kontaktfläche (57) des Spannockens (20) so geneigt ist, daß mindestens auf einem Teil der dem Vorderende (3) des Gehäuses (2) zugewandten Hälfte der Wendellänge Flächenkontakt zu der Gleitfläche (22) der Wendel (21) besteht.

6. Blutentnahmegerät nach Anspruch 5, bei welchem an dem dem Hinterende (16) des Gehäuses (2) zugewandten Ende der Wendel (21) ein rampenförmiger Startabschnitt (58) vorgesehen ist, dessen Steigung der Neigung der Kontaktfläche (57) entspricht.

7. Blutentnahmegerät nach einem der Ansprüche 2 bis 6, bei welchem die Wendel (21) an einer Spannhülse (59) ausgebildet ist, die einen Teil des Lanzettenantriebsrotors (13) bildet und deren der Austrittsöffnung (5) zugewandtes vorderes Ende den Lanzettenhalter (8) umgibt.

8. Blutentnahmegerät nach einem der Ansprüche 2 bis 7, bei welchem an dem Lanzettenantriebsrotor (13) zwei parallel verlaufende Wendeln (21) vorgesehen und mit dem Spannknopf (2) zwei mit diesem längs des Spannweges (23) bewegliche Spannocken (20) verbunden sind, die zum Spannen des Lanzettenantriebs (12) jeweils auf einer der Gleitflächen (22) der Wendeln (21) gleiten.

9. Blutentnahmegerät nach einem der Ansprüche 2 bis 8, bei welchem das Material der Gleitfläche (22) der Wendel (21) ein Polyacetal, insbesondere ein Polyoxymethylen (POM) enthält.

10. Blutentnahmegerät nach einem der Ansprüche 2 bis 9, bei welchem das Material der Kontaktfläche (57) des Spannockens (20) ein Styrol-Acrylnitril-Copolymer (SAN) enthält.

11. Blutentnahmegerät nach einem der vorhergehenden Ansprüche, bei welchem die Ausgangsseite (26) des doppelseitigen Drehschiebegetriebes (25) eine Kurvensteuerung (28) mit einer eine Steuerkurve (30) bildenden Ausnehmung (32) des Lanzettenantriebsrotors (13) aufweist, in die ein passender Steuerzapfen (31) eingreift, der mit dem Lanzettenhalter (8) verbunden ist, wobei mindestens ein Teil der Einstichbewegung durch die während der Rotationsbewegung des Lanzettenantriebsrotors (13) stattfindende Rotationsbewegung der Steuerkurve (30) relativ zu dem Steuerzapfen (31) bestimmt wird, bei der der Steuerzapfen (31) die durch die Ausnehmung (32) gebildete Steuerkurve (30) abfährt.

12. Blutentnahmegerät nach Anspruch 11, bei welchem auch die Rückführbewegung des Lanzettenhalters (8) durch die Rotationsbewegung der Steuerkurve (30) relativ zu dem Steuerzapfen (31) bestimmt wird.

13. Blutentnahmegerät nach einem der Ansprüche 11 oder 12, bei welchem zwei Steuerzapfen (31) mit dem Lanzettenhalter (8) verbunden sind, die in zwei verschiedene Ausnehmungen (61,62) des Lanzettenantriebsrotors (13) eingreifen, wobei in jeder Phase der Rotationsbewegung des Lanzettenantriebsrotors (13) eine der Ausnehmungen (61,62) die Führung des einen Steuerzapfens (31) nach hinten und die andere Ausnehmung die Führung des anderen Steuerzapfens (31) nach vorne bildet.

14. Blutentnahme-Kit zur Entnahme von Blut für Diagnosezwecke, umfassend ein Blutentnahmegerät (1) nach einem der vorhergehenden Ansprüche und Lanzetten (7), die zur Halterung in dessen Lanzettenhalter (8) ausgebildet sind.

## Claims

1. Blood lancet device for withdrawing blood for diagnostic purposes comprising
an elongated housing (2), the front end (3) of which having an exit opening (5) for the tip (6) of a lancet (7),
a lancet holder (8) for holding the lancet (7), the lancet holder (8) being movable in the housing (2) in the direction of its main axis (A) along a predetermined puncturing path,
a lancet guide (49) for guiding the lancet holder (8) along the predetermined puncturing path and
a lancet drive (12) having an elastic drive spring (15) which can be locked by a locking device (19) in the loaded state of the drive spring (15), the lancet drive (12) converting after triggering of the locking device the tension-release movement of the drive spring (15) into a puncturing motion to move the lancet (7), held in the lancet holder (8), at high speed along the predetermined puncturing path in the puncturing direction (10) until its tip (6) emerges from the exit opening (5) to produce a wound in a body part adjacent the exit opening (5),
**characterized in that**
a double sided rotary/translatory transmission (25) is provided in the housing (2),
a) the input side (24) of the rotary/translatory transmission (25) converting the motion of a loading button (17), which projects out of the housing (2) at its rear end (16) and moves along a linear loading path (23), into a rotational motion of a lancet drive rotor (13), the lancet drive rotor (13) being rotated about a rotational axis running parallel to the axis of the device (A) to load the lancet drive rotor (13) by tensioning the drive spring (15) and
b) the output side (26) of the rotary/translatory transmission (25) converting after triggering of the lancet drive a rotational motion of the lancet drive rotor driven by the drive spring into the puncturing motion in the direction of the main axis.

2. Blood lancet device according to claim 1, wherein the input side (24) of the two-sided rotary/translatory transmission (25) comprises a helical path (21) provided on the lancet drive rotor (13) and a loading cam (20) connected to the loading button (17) for motion therewith along the loading path (23) and sliding via a loading cam (20) contact surface (57) along a slide surface (22) of the helical path (21).

3. Blood lancet device according to claim 2, wherein the slope (α) of the helical path (21) increases, at least in sections of its length in the direction towards the front end (3) of the housing (2).

4. Blood lancet device according to claim 3, wherein the slope (α) varies in such a fashion that the loading force required for moving the loading button (17) during loading of the lancet drive (12) is substantially constant, at least in sections of the loading path (23).

5. Blood lancet device according to any one of claims 2 through 4, wherein the contact surface (57) of the loading cam (20) is slanted for area contact with the slide surface (22) of the helical path (21) along at least a portion of that half of the length of the helical path which is closer to the front end (3) of the housing (2).

6. Blood lancet device according to claim 5, wherein a ramped starting section (58) is provided on the end of the helical path (21) which is closer to the rear end (16) of the housing (2), the starting section (58) having a slope corresponding to the slope of the contact surface (57).

7. Blood lancet device according to any one of claims 2 through 6, wherein the helical path (21) is formed on a loading sleeve (59) constituting a portion of the lancet drive rotor (13), wherein the front end of the loading sleeve (59) facing the exit opening (5) surrounds the lancet holder (8).

8. Blood lancet device according to any one of claims 2 to 7, wherein the lancet drive rotor (13) comprises two parallel helical paths (21) and two loading cams (20) are connected to the loading button (17) for movement with the loading button (17) through the loading path (23), each of the loading cams (20) sliding during loading of the lancet drive (12) along a slide surface (22) of one of the helical paths (21).

9. Blood lancet device according to any one of the claims 2 to 8, wherein the material forming the slide surface (22) of the helical path (21) comprises a polyacetal, preferentially a polyoxymethylene (POM).

10. Blood lancet device according to any one of claims 2 to 9, wherein the material forming the contact surface (57) of the loading cam (20) comprises a styrene-acrylnitril-copolymer (SAN).

11. Blood lancet device according to any one of the preceding claims, wherein the output side (26) of the two-sided rotary/translatory transmission (25) comprises a cam drive mechanism (28), the cam drive mechanism (28) comprising a recess (32) in the lancet drive rotor (13) defining a cam guide (30), and a control pin (31) engaging into the recess (32) and connected to.the lancet holder (8), wherein at least a portion of the puncturing motion is defined by the rotational motion of the cam guide (30) relative to the control pin (31) during the rotational motion of the lancet drive rotor (13), during which the control pin (31) travels along the recess (32) defining the cam guide (30).

12. Blood lancet device according to claim 11, wherein the return motion of the lancet holder (8) is also defined by the rotational motion of the cam guide (30) relative to the control pin (31).

13. Blood lancet device according to any one of claims 11 or 12, wherein two control pins (31) are connected to the lancet holder (8) and engage into two differing recesses (61, 62) in the lancet drive rotor (13), wherein, during each phase of the rotational motion of the lancet drive rotor (13), one of the recesses (61, 62) guides one control pin (31) in rearward direction and the other recess guides the other control pin (31) in forward direction.

14. Kit for withdrawing blood for diagnostic applications, comprising a blood lancet device (1) according to any one of the preceding claims and lancets (7) adapted to be held in the lancet holder (8) thereof.

## Revendications

1. Instrument de prélèvement du sang pour prélever du sang à des fins diagnostiques, comprenant
un logement (2) s'étendant en direction longitudinale, à l'extrémité avant (3) duquel on prévoit une ouverture de sortie (5) pour la pointe (6) d'une lancette (7),
un support de lancette (8) mobile dans le logement (2) en direction de l'axe principal (A) de ce dernier, le long d'un tronçon de piqûre prédéfini, destiné à maintenir la lancette (7) ;
un guidage de lancette (49) pour guider le support de lancette (8) le long du tronçon de piqûre prédéfini ;
un entraînement de lancette (12) comprenant un ressort d'entraînement élastique (15), qui peut être verrouillé, à l'état tendu du ressort d'entraînement (15), à l'aide d'un dispositif de verrouillage (19) et par lequel, après le relâchement du dispositif de verrouillage, le mouvement de détente du ressort d'entraînement (15) est transformé en un mouvement de piqûre au cours duquel la lancette (7) maintenue par le support de lancette (8) est mise en mouvement à une vitesse élevée dans une direction de piqûre (10) le long du tronçon de piqûre prédéfini, jusqu'à ce que sa pointe (6) ressorte par l'ouverture de sortie (5) pour générer une blessure dans une partie du corps adjacente à l'ouverture de sortie (5),
**caractérisé en ce que**
dans le logement (2), on prévoit un mécanisme de transmission (25) du type à chemise rotative,
a) sur le côté entrée (24) duquel le mouvement d'un bouton de tension (17), faisant saillie par rapport au logement (2), à l'extrémité arrière (16) de ce dernier, ledit mouvement s'étendant le long d'un tronçon de tension linéaire (23), est transformé en un mouvement de rotation de rotor d'entraînement de lancette (13) qui effectue une rotation autour d'un axe de rotation s'étendant parallèlement à l'axe (A) de l'instrument, dans le but d'exercer une tension sur le rotor d'entraînement de lancette (13) à l'encontre de la force exercée par le ressort d'entraînement (15), et
b) sur le côté sortie (26) duquel, après le relâchement de l'entraînement de lancette, un mouvement de rotation du rotor d'entraînement de lancette, sous la commande du ressort d'entraînement, est transformé en mouvement de piqûre dans la direction de l'axe principal.

2. Instrument de prélèvement du sang selon la revendication 1, dans lequel le côté entrée (24) du mécanisme de transmission bilatéral (25) du type à chemise rotative est réalisé à l'aide d'une hélice (21) prévue contre le rotor d'entraînement de lancette (13) et à l'aide d'une came de tension (20) reliée au bouton de tension (17), mobile avec ce dernier le long du tronçon de tension (23) et apte à glisser sur une surface de glissement (22) de l'hélice (21) via une surface de contact (57).

3. Instrument de prélèvement du sang selon la revendication 2, dans lequel le pas (α) de l'hélice (21) augmente, au moins par section, sur la longueur de ladite hélice, en direction de l'extrémité avant (3) du logement (2).

4. Instrument de prélèvement du sang selon la revendication 3, dans lequel la variation du pas (α) est telle que la force de tension, qui doit s'exercer sur le bouton de tension (17), requise pour la mise sous tension de l'entraînement de lancette (12), est essentiellement constante, au moins par section, sur le tronçon de tension (23).

5. Instrument de prélèvement du sang selon l'une quelconque des revendications 2 à 4, dans lequel la surface de contact (57) de la came de tension (20) présente une inclinaison telle qu'au moins sur une partie de la moitié de la longueur de l'hélice tournée vers l'extrémité avant (3) du logement (2), on obtient un contact, du type s'étendant sur toute la surface, avec la surface de glissement (22) de l'hélice (21).

6. Instrument de prélèvement du sang selon la revendication 5, dans lequel, à l'extrémité de l'hélice (21) tournée vers l'extrémité arrière (16) du logement (2), on prévoit une section de démarrage (58) en forme de rampe dont la pente correspond à l'inclinaison de la surface de contact (57).

7. Instrument de prélèvement du sang selon l'une quelconque des revendications 2 à 6, dans lequel l'hélice (21) est réalisée contre un manchon de tension (59) qui forme une partie du rotor d'entraînement de lancette (13) et dont l'extrémité avant tournée vers l'ouverture de sortie (5) entoure le support de lancette (8).

8. Instrument de prélèvement du sang selon l'une quelconque des revendications 2 à 7, dans lequel, contre le rotor d'entraînement de lancette (13), on prévoit deux hélices (21) s'étendant en parallèle, deux cames de tension (20), mobiles avec le bouton de tension (2) le long du tronçon de tension (23), étant reliées audit bouton de tension (2), lesdites deux cames, pour la mise sous tension de l'entraînement de lancette (12) glissant respectivement sur une des surfaces de glissement (22) des hélices (21).

9. Instrument de prélèvement du sang selon l'une quelconque des revendications 2 à 8, dans lequel la matière de la surface de glissement (22) de l'hélice (21) contient un polyacétal, en particulier un polyoxyméthylène (POM).

10. Instrument de prélèvement du sang selon l'une quelconque des revendications 2 à 9, dans lequel la matière de la surface de contact (57) de la came de tension (20) contient un copolymère de styrène-acrylonitrile (SAN).

11. Instrument de prélèvement du sang selon l'une quelconque des revendications précédentes, dans lequel le côté sortie (26) du mécanisme de transmission bilatéral (25) du type à chemise rotative présente une commande de came (28) comprenant un évidement (32) du rotor d'entraînement de lancette (13), formant une came de commande (30), dans laquelle vient s'insérer un tourillon de commande correspondant (31) qui est relié au support de lancette (8), au moins une partie du mouvement de piqûre étant déterminée par le mouvement de rotation de la came de commande (30) par rapport au tourillon de commande (31), en vigueur lors du mouvement de rotation du rotor d'entraînement de lancette (13), mouvement au cours duquel le tourillon de commande (31) emmène la came de commande (30) formée par l'évidement (32).

12. Instrument de prélèvement du sang selon la revendication 11, dans lequel, également le mouvement de retour du support de lancette (8) est déterminé par le mouvement de rotation de la came de commande (30) par rapport au tourillon de commande (31).

13. Instrument de prélèvement du sang selon l'une quelconque des revendications 11 ou 12, dans lequel deux tourillons de commande (31) sont reliés au support de lancette (8), les deux tourillons venant s'insérer dans deux évidements différents (61, 62) du rotor d'entraînement de lancette (13), un des évidements (61, 62) formant le guidage d'un des tourillons de commande (31) vers l'arrière et l'autre évidement formant le guidage de l'autre tourillon de commande (31) vers l'avant, lors de chaque phase du mouvement de rotation du rotor d'entraînement de lancette (13).

14. Nécessaire de prélèvement du sang pour prélever du sang à des fins diagnostiques, comprenant un instrument de prélèvement du sang (1) selon l'une quelconque des revendications précédentes et des lancettes (7) qui sont réalisées de façon à être maintenues dans le support de lancette (8) de l'instrument en question.
